# EUROPEAN PATENT APPLICATION

(11) **EP 2 308 464 A1**
(43) Date of publication of application: **13.04.2011**
(21) Application number: 10186621.8
(22) Date of filing: 05.10.2010
(51) Int. Cl.: A61K 9/00, A61K 31/428

(54) **Orally disintegrating compositions of pramipexole**

(30) Priority: 06.10.2009 TR 200907554
(71) Applicant: Sanovel Ilac Sanayi ve Ticaret A.S., 34398 Istanbul (TR)
(72) Inventor: Toksoz, Ahmet, 34398 Istanbul (TR); Yelken, Gülay, 34398 Istanbul (TR); Turp, Hasan Ali, 34398 Istanbul (TR); Turkzilmaz, Ali, 34398 Istanbul (TR); Cifter, Umit, 34398 Istanbul (TR)
(74) Representative: Sevinç, Erkan

(57) **Abstract**

The present invention is related to an orally disintegrating composition comprising pramipexole or a pharmaceutically acceptable salt, solvate and hydrate thereof and at least one pharmaceutically acceptable excipient, for their preparation and use thereof.

## Description

### Technical Aspect

The present invention is related to an orally disintegrating composition comprising pramipexole or a pharmaceutically acceptable salt, solvate and hydrate thereof and at least one pharmaceutically acceptable excipient, for their preparation and use thereof.

### Background of the Invention

Pramipexole is a nonergot dopamine agonist. The chemical name of pramipexole is (S)-2-amino-4,5,6,7-tetrahydro-6-(propylamino)benzothiazole and its chemical structure is shown in the Formula 1.

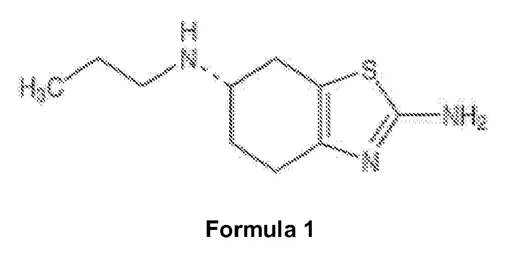

Pramipexole conventional tablet forms are marketed under the name of Mirapex^{®} and are indicated to be taken 3 times a day for oral administration and contain 0.125 mg, 0.25 mg, 0.5 mg, 1 mg, or 1.5 mg of pramipexole dihydrochloride monohydrate as active ingredient. Mirapex^{®} tablets are indicated for the treatment of the signs and symptoms of idiopathic Parkinson's disease and moderate-to-severe primary Restless Legs Syndrome (RLS).

Pramipexole and processes for preparing it and the treatment of schizophrenia and Parkinson's disease are firstly described in EP0186087B1.

Parkinson's disease is a progressive, neurodegenerative disorder of the nervous system that affects the neurons in the part of the brain that controls mobility and muscle movement and causes symptoms such as tremor, muscle rigidity, bradykinetic movements, difficulty in walking, problems with balance and coordination.

Further, Restless Legs Syndrome (RLS) is a fairly common sensorimotor disorder, characterized by an irresistible urge to move one's body to stop uncomfortable or odd sensations. RLS typically gives the individual who suffers from it, an unpleasant sensation in the legs at rest, causing what is often described as an irresistible desire to move, leading to significant discomfort. It most commonly affects the legs, but can also affect the arms.

Such type of movement disorders like restless legs syndrome and Parkinson's disease further complicate the administration of medication to these patients especially to elderly population. Most commonly used solid dosage forms for oral administration such as tablets or capsules are not suitable for this population as they may have difficulty in swallowing. With liquid dosage forms, administration and stability of the drug and the dosage form can be a major concern. An ideal solid dosage form for these patients includes a formulation that simply melts in the mouth without any extra effort such as an orally disintegrating composition.

In addition to these problems in prior art, some pediatric, geriatric, and psychiatric patient populations exhibit "cheeking" behavior (i.e., holding the oral dosage form in the cheek) to avoid swallowing the medication. Accordingly, orally disintegrating compositions would be desirable to improve patient compliance, particularly among elderly patients, because orally disintegrating compositions are easier to swallow and prevent "cheeking".

Besides, another problem for conventional solid dosage forms is their inconvenience for bedridden or busy and travelling patients, in case these patients may not have easy access to water. Thus, orally disintegrating compositions represent an alternative for such patients and provide a better patient compliance with recommended pharmaceutical therapies.

In prior art, there are many patents of conventional tablet formulations of pramipexole dihydrochloride monohydrate and use thereof such as EP0989850B1, EP1414446B1, EP1416930A1, EP1940398A1, EP2086536A1 and WO 2008/145252 A1; but none of them include an orally disintegrating composition of pramipexole dihydrochloride monohydrate.

Thus, a need rises and the present invention discloses formulations for orally disintegrating compositions of pramipexole or a pharmaceutically acceptable salt, solvate and hydrate thereof which overcome above described problems and which further provide the advantageous property of allowing the active medicament to disintegrate or dissolve rapidly in the oral cavity which have a pleasant mouth feel and good mechanical strength, enough to be processed in high speed tableting machines and shipped in low cost packages.

### Description of the invention

The present invention provides a novel orally disintegrating composition comprising pramipexole or a pharmaceutically acceptable salt, solvate and hydrate thereof and at least one pharmaceutically acceptable excipient which overcomes the above described problems in prior art and have additive advantages over them.

The main object of the present invention is to provide orally disintegrating compositions offering various low dosage strengths of pramipexole or a pharmaceutically acceptable salt, solvate and hydrate thereof, capable of providing the patients an increased convenience, easy administration, rapid and reliable disintegration, easy dosage adjustment and better control of movement disorder disease symptoms, with using adequate excipients and improved processes.

Another object of the present invention is to obtain orally disintegrating compositions of pramipexole or a pharmaceutically acceptable salt, solvate and hydrate thereof wherein the composition disintegrates in oral cavity in less than 60 seconds, preferably in less than 30 seconds.

In a preffered embodiment, a pharmaceutically acceptable salt, solvate and hydrate of pramipexole is in the form of dihydrochloride monohydrate salt.

A further object of this invention is to provide bioavailable and stable orally disintegrating compositions of pramipexole or a pharmaceutically acceptable salt, solvate and hydrate thereof, throughout the shelf-life. According to this object, in this invention a pramipexole orally disintegrating composition which is comparable with the existing conventional solid dosage forms such as tablets or capsules is provided, however the unexpected benefits are found with oral disintegration. Because, presentation of pramipexole in conventional solid or liquid oral dosage forms, having their own limitations, are not ideal for use in pediatric or geriatric patients or in patients suffering from various movement disorders like Parkinson's Disease or RLS. According to this embodiment, the present invention is directed to a method of improving patient compliance with the administration of a drug such as pramipexole, comprising administering an orally disintegrating composition comprising a therapeutically effective amount of pramipexole dihydrochloride monohydrate in an amount of 0.01 to 5% by weight of total composition; preferably it is 0.1 to 2.0% by weight of total composition.

In another embodiment, the amount of pramipexole dihydrochloride monohydrate is 0.01 mg to 5.0 mg; preferably it is 0.1 mg to 2.0 mg.

Another object of the present invention is an orally disintegrating composition of pramipexole dihydrochloride monohydrate further comprising a dispersing agent.

In one embodiment, when the amount of dispersing agent is present in an amount of 1 to 90 % by weight of the total orally disintegrating composition, preferably from 5 to 50% by weight, more preferably from 15 to 40 % by weight of the total composition, said amount makes it possible to significantly improve compressibility, reduce friability and achieve a substantial reduction in disintegration time. Higher quantities may have negative mechanichal strength of the formula and lower quantities may worsen the disintegration time.

Accoriding to the invention suitable dispersing agent is selected from the group comprising calcium silicate, magnesium silicate, magnesium aluminium trisilicate, amorphous silica, magnesium carbonate heavy and the like and mixtures thereof. Preferably the dispersing agent selected for this invention is calcium silicate, wherein the calcium silicate has a median particle size of less than 50 micron, preferably the particle size is less than 20 micron, more preferably it is less than 10 micron.

Another object of the present invention is to develop orally disintegrating compositions having optimal mechanical strength. The present invention addresses this need and discloses formulations that rapidly disintegrate in the oral cavity. These tablet compositions have a pleasant mouth feel and good mechanical strength. These tablets are robust (e.g., low friability, adequate hardness) enough to be processed in high speed tableting machines and shipped in low cost packages, and at the same time retain rapid disintegration or dissolution properties. These orally disintegrating compositions are bioavailable in correspondence with the conventional solid dosage formulations and stable trhoughout the shelf-life.

According to another object of the present invention, the hardness of the orally disintegration tablet is between 5 N to 100 N, preferably it is between 20 N to 45 N; and the firiabilite of the orally disintegration tablet is less than 1.0 %.

It is known that, to develop orally disintegrating compositions are difficult because of several different reasons. A satisfied orally disintegrating dosage form needs to meet number of requirements. Firstly, it has to disintegrate in the oral cavity rapidly. Moreover, a premature release in the mouth could also lead to problems due to the often unpleasant taste of the active ingredient. Besides, these compositions should be very porous and should not be very hard. These porous compositions tend to be very sensitive to humidity. As a consequence, they may have some stability problems.

However, in order to be pharmacologically acceptable, orally disintegrating compositions must be palatable, e.g. have acceptable organoleptic properties such as good taste and mouthfeel, because orally disintegrating compositions are designed to disintegrate in the oral cavity of the patient rapidly without remaning substantial amounts of the active ingredient. In addition, the orally disintegrating formulations must also provide acceptable pharmacokinetics and bioavailability to provide the desired therapeutic effect. Conversely, components of the formulation that promote rapid release may result in undesirable taste or mouthfeel properties. Finally, any orally disintegrating composition with suitable organoleptic and pharmacokinetic properties must also be manufactured at commercially useful rates and yields.

Accordingly, an acceptable orally disintegrating composition must balance these contradictory characteristics in order to provide a palatable (e.g., taste-masked), rapid disintegrating composition with acceptable pharmacokinetics.

To fulfill all these requirements the formulation for a specific drug needs to be adapted in particular by a careful selection of the excipients used. However, the excipients selected may lead to formulations which are not bioavailable to the corresponding conventional dosage forms. Thus, they have to be chosen very carefully.

The orally disintegrating compositions of this invention further comprising at least one pharmaceutically acceptable excipient selected from the group comprising super disintegrants, diluents and/or fillers, binders and/or adhesives, lubricants, glidants, sweeteners, flavouring agents, coloring agents and preservatives.

Suitable super disintegrants may comprise but not limited to crospovidone, croscarmellose sodium, low-substituted hydroxypropylcellulose, sodium starch glycollate, pregelatinized starch and the like and mixtures thereof. Preferably the super disintegrant selected for this invention is crospovidone.

It has unexpectedly been found that the selected ratio range of pramipexole dihydrochloride monohydrate salt to super disintegrants, particularly crospovidone provides an orally disintegrating composition which avoids the afore-mentioned disadvantages of the orally disintegrating compositions of the prior art.

Crospovidone, has physical and chemical properties that make it ideal for constituting the appropriate super disintegrant for this invention. Because crospovidone particles have a very different appearance from those of the other super disintegrants. Crospovidone particles seem to consist of aggregates of smaller particles that are fused together. This aggregation gives crospovidone a spongy, highly porous appearance and it swells very little, yet takes water into its network quite rapidly. This helps crospovidone to dissolve easily and quickly in a little amount of water or saliva and makes its disintegrating rate much faster than other related excipients.

It has unexpectedly found that in this orally disintegrating composition having a weight ratio of pramipexole to super disintegrants, particularly crospovidone, in the range of between 1:100 and 50:1 (w/w) has a synergistic effect over the disintegration time. Preferably the range is between preferebly 1:50 and 1:1 (w/w), more preferably the range is between 1:45 and 1:20 (w/w).

In one embodiment, the amount of crospovidone is present 0.1 to 30% by weight of total composition; preferably it is 1 to 15% by weight of total composition.

Surprisingly we have found that when the weight ratio of crospovidone to calcium silicate in the range of between 1:10 and 10:1 (w/w), it has synergistic effect over the mechanical strength (such as; hardness and friability) of the orally disintegrating composition. Preferably the range is between 1:5 and 5:1 (w/w), more preferably it is in the range of between 1:5 and 1:1 (w/w).

Suitable diluents and/or fillers may comprise but not limited to mannitol, spray-dried mannitol; polysaccharides such as microcrystalline cellulose; dibasic calcium phosphate dihydrate, lactose, sugars, sorbitol; mixture of microcrystalline cellulose and guar gum (Avicel CE-15); mixture of mannitol, polyplasdone and syolid (Pharmaburst); mixture of mannitol, crospovidone and polyvinyl acetate (Ludiflash); isomalt, Panexcea, F-Melt, sucrose, inorganic salts such as calcium salts; magnesium carbonate heavy and the like and mixtures thereof; preferably the diluent and/or filler is mannitol and/or microcrystalline cellulose.

Suitable binders and/or adhesives may comprise but not limited to polyvinylpyrrolidone (povidone), gelatin, sugars, glucose, natural gums, gums, synthetic celluloses, polymethycrylate, cellulose derivatives such as hydroxypropyl methyl cellulose, hydroxypropyl cellulose, carboxy methyl cellulose, methyl cellulose and the like and mixtures thereof; preferably the binder is polyvinylpyrrolidone.

Suitable lubricants may comprise but not limited to sodium stearyl fumarate, magnesium stearate, polyethylene glycol, stearic acid, metal stearates, boric acid, sodium chloride benzoate and acetate, sodium or magnesium lauryl sulfate and the like and mixtures thereof; preferably the lubricant is sodium stearyl fumarate.

Although the preferred lubricant is sodium stearyl fumarate, other less hydrophobic lubricants may be used to counter the hydrophobicity in certain cases such as a combination of sodium stearyl fumarate with magnesium stearate, sodium lauryl sulfate or hydrogenated vegetable oils.

Suitable glidants may comprise but not limited to colloidal silicon dioxide, talc, aluminium silicate and the like and mixtures thereof.

The orally disintegrating compositions of this invention also comprise sweeteners and flavouring agents to improve patient compliance.

Suitable sweeteners may comprise but not limited to sucralose, acesulfame-K, aspartame, saccharin or its sodium and calcium salts, sodium cyclamate, sucrose, fructose, glucose, sorbitol and the like and mixtures thereof; preferably the sweetner is sucralose.

Suitable flavouring agents may comprise but not limited to fruit flavours such as orange, cherry, strawberry, banana, wild cherry, lemon; cardamom, anise, peppermint, menthol, vanillin and ethyl vanillin and the like and mixtures thereof; preferably the flavouring agent is fruit flavour such as orange.

Suitable coloring agents are selected from the group comprising Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow and the like and mixtures thereof; preferably the coloring agent is iron oxide yellow.

Suitable preservatives may comprise but not limited to methyl paraben and propyl paraben and their salts (such as sodium, potassium), sodium benzoate, citric acid, benzoic acid, butylated hydroxytoluene and butylated hydroxyanisole and the like and mixtures thereof. In one aspect, the preservative content may present in an amount of about from 0,01 to 5%, preferably about from 0.5 to 2% by weight of total composition.

Optionally, a humidity absorbent agent may be added, such as precipitated silica in a proportion from 0.01 to 1% in weight of the total weight of the tablet, which may counteract the hydrophobicity of the active ingredient and improve the fluidity of the mixture.

The orally disintegrating compositions of this invention include tablets, sachets, minitablets, multilayer and multicoated tablets, pellets or powders which can be formulated in accordance with methods that are standard in the art. It is preferably in the form of tablets.

In a further aspect, the present invention shows that it is possible to have a significant influence on the disintegration rate of the tablet by modifying the dimensions and shape of the tablet. In general, as the tablet becomes thinner and have higher porosity, the orally disintegrating composition will be weakened faster when it contacts with saliva, because the disintegration process is produced after wetting all the surface of the tablet via capillary action. Also, any shape which maximizes the contact surface with the saliva may produce a significant reduction in disintegration time.

The preferred shape of the orally disintegrating tablet composition of this invention may have a shape of a disk, circle, round, sphere, donut, bar, polygon, ellipse and the like.

In this present invention, to minimize the disintegration time and maximise the mechanical resistance of the tablets of this invention, this orally disintegrating tablet composition has been designed, made up of the following:
a. 0.01 to 5.0% by weight of pramipexole dihydrochloride monohydrate,
b. 5.0 to 90% by weight of mannitol
c. 1.0 to 90% by weight of calcium silicate,
d. 0.1 to 25% by weight of polivinylpyrrolidone
e. 1.0 to 30 % by weight of crospovidone
f. 0.1 to 2% by weight of sucralose
g. 0.1 to 5% by weight of orange flavour
h. 0.1 to 10 % by weight of sodium stearyl fumarate.

In one embodiment, the orally disintegrating compositions of this invention are suitable for the treatment of Parkinsonism or parkionson's disease and complications or disorders associated therewith, schizophrenia and restless leg syndrome.

In another embodiment, the orally disintegrating compositions according to the present invention may further comprise one or more other active ingredients such as L-dopa.

In one embodiment, the orally disintegrating compositions of the present invention may be prepared by conventional technology well known to those skilled in the art such as direct compression, dry granulation, wet granulation and the like. The manufacturing process is preferably wet granulation.

The preferred process for preparing the orally disintegrating compositions of the invention comprises the following steps:
a. dissolving pramipexole dihydrochloride monohydrate and polivinylpyrrolidone in water and/or alcohol to form an aqueous pramipexole dihydrochloride monohydrate solution,
b. while the solution is mixing, mannitol and half part of crospovidone is added and blended in a high-shear granulator to form granules,
c. sieving and drying the wet granules and sieving the dried granules,
d. adding calcium silicate, sucralose, the rest of the crospovidone and flavour and mixing them,
e. adding sodium stearyl fumarate to this mixture and blending them until obtaining a homogenous powder mixture,
f. compressing the blended mixture to form tablets.

Another preffered process for preparing the orally disintegrating compositions of the invention comprises the following steps:
a. dissolving pramipexole dihydrochloride monohydrate and polivinylpyrrolidone in water and/or alcohol to form an aqueous pramipexole dihydrochloride monohydrate solution,
b. mixing mannitol and half part of crospovidone in a high-shear granulator,
c. spraying the pramipexole dihydrochloride monohydrate solution to the blended powder mixture of mannitol and crospovidone ,
d. sieving and drying the wet granules and sieving the dried granules,
e. adding calcium silicate, sucralose, the rest of the crospovidone and flavour and mixing them,
f. adding sodium stearyl fumarate to this mixture and blending them until obtaining a homogenous powder mixture,
g. compressing the blended mixture to form tablets.

The orally disintegrating compositions of the present invention may also be prepared alternatively by other technologies such as spray drying, freeze drying, floss formation, molding, Zydis^{®} technology, Flashtab technology, OraSolv^{®} technology, DuraSolv^{®} technology, Wowtab^{™} (With Out Water quick-dissolve tablet) technology, Lyoc^{™}, Shearform^{™}, Quicksolv^{®}, Efvdas^{®} and the like.

In one aspect, the orally disintegrating compositions according to present invention further comprise one or more other active ingredients, such as L-dopa; these ingredients can be added in any step of the manufacturing process as described above.

This invention is further defined by reference to the following examples. Although the examples are not intended to limit the scope of the present invention, it should be considered in the light of the description detailed above. It will be apparent to those skilled in the art that many modifications, both to materials and methods, may be practiced without departing from the scope of the invention.

### Example 1

| **Ingredients** | **Amount (mg)** |
|---|---|
| Pramipexole dihydrochloride monohydrate | 0.125 |
| Mannitol | 30.000 |
| Calcium silicate | 14.925 |
| Polyvinylpyrollidone (Povidone K-30) | 0.520 |
| Crospovidone (Kollidon CL-SF) | 5.250 |
| Sucralose | 0.105 |
| Orange flavour | 0.525 |
| Sodium stearyl fumarate | 1.050 |
| Total tablet weight | 52.50 |

### Example 2

| **Ingredients** | **Amount (mg)** |
|---|---|
| Pramipexole dihydrochloride monohydrate | 0.250 |
| Mannitol | 29.880 |
| Calcium silicate | 14.920 |
| Polyvinylpyrollidone (Povidone K-30) | 0.520 |
| Crospovidone (Kollidon CL-SF) | 5.250 |
| Sucralose | 0.105 |
| Orange flavour | 0.525 |
| Sodium stearyl fumarate | 1.050 |
| Total tablet weight | 52.50 |

### Example 3

| **Ingredients** | **Amount (mg)** |
|---|---|
| Pramipexole dihydrochloride monohydrate | 0.50 |
| Mannitol | 59.76 |
| Calcium silicate | 29.84 |
| Polyvinylpyrollidone (Povidone K-30) | 1.04 |
| Crospovidone (Kollidon CL-SF) | 10.50 |
| Sucralose | 0.21 |
| Orange flavour | 1.05 |
| Sodium stearyl fumarate | 2.10 |
| Total tablet weight | 105.00 |

### Example 4

| **Ingredients** | **Amount (mg)** |
|---|---|
| Pramipexole dihydrochloride monohydrate | 1.00 |
| Mannitol | 119.52 |
| Calcium silicate | 59.68 |
| Polyvinylpyrollidone (Povidone K-30) | 2.08 |
| Crospovidone (Kollidon CL-SF) | 21.00 |
| Sucralose | 0.42 |
| Orange flavour | 2.10 |
| Sodium stearyl fumarate | 4.20 |
| Total tablet weight | 210.00 |

### Example 5

| **Ingredients** | **Amount (mg)** |
|---|---|
| Pramipexole dihydrochloride monohydrate | 1.25 |
| Mannitol | 149.40 |
| Calcium silicate | 74.60 |
| Polyvinylpyrollidone (Povidone K-30) | 2.60 |
| Crospovidone (Kollidon CL-SF) | 26.25 |
| Sucralose | 0.525 |
| Orange flavour | 2.625 |
| Sodium stearyl fumarate | 5.25 |
| Total tablet weight | 262.50 |

### Example 6

| **Ingredients** | **Amount (mg)** |
|---|---|
| Pramipexole dihydrochloride monohydrate | 1.50 |
| Mannitol | 179.28 |
| Calcium silicate | 89.52 |
| Polyvinylpyrollidone (Povidone K-30) | 3.12 |
| Crospovidone (Kollidon CL-SF) | 31.50 |
| Sucralose | 0.63 |
| Orange flavour | 3.15 |
| Sodium stearyl fumarate | 6.30 |
| Total tablet weight | 315.00 |

### Example 7

| **Ingredients** | **Amount (mg)** |
|---|---|
| Pramipexole dihydrochloride monohydrate | 0.125 |
| Mannitol | 20.060 |
| Microcrystalline cellulose | 9.950 |
| Calcium silicate | 14.920 |
| Polyvinylpyrollidone | 0.520 |
| Crospovidone | 5.250 |
| Sucralose | 0.105 |
| Orange flavour | 0.520 |
| Sodium stearyl fumarate | 1.050 |
| Total tablet weight | 52.50 |

### Example 8

| **Ingredients** | **Amount (mg)** |
|---|---|
| Pramipexole dihydrochloride monohydrate | 0.125 |
| Mannitol | 20.04 |
| Microcrystalline cellulose | 9.95 |
| Avicel CE-15 | 4.72 |
| Calcium silicate | 10.22 |
| Polyvinylpyrollidone | 0.520 |
| Crospovidone | 5.250 |
| Sucralose | 0.105 |
| Orange flavour | 0.520 |
| Sodium stearyl fumarate | 1.050 |
| Total tablet weight | 52.50 |

### Example 9

| **Ingredients** | **Amount (mg)** |
|---|---|
| Pramipexole dihydrochloride monohydrate | 0.125 |
| Magnesium carbonate heavy | 21.00 |
| Mannitol | 23.93 |
| Polyvinylpyrollidone | 0.520 |
| Crospovidone | 5.250 |
| Sucralose | 0.105 |
| Orange flavour | 0.520 |
| Sodium stearyl fumarate | 1.050 |
| Total tablet weight | 52.50 |

The formulations of these examples are manufactured according to the processes described above in the description and tablet formulations may further comprise coloring agents.

## Claims

1. An orally disintegrating composition comprising pramipexole or a pharmaceutically acceptable salt, solvate and hydrate thereof and at least one pharmaceutically acceptable excipient, wherein at least one pharmaceutical excipient is a dispersing agent which is present in an amount of 1 to 90% by weight of total composition.

2. The orally disintegrating composition according to claim 1, wherein the composition disintegrates in oral cavity in less than 60 seconds, preferably in less than 30 seconds

3. The orally disintegrating composition according to claims 1 and 2, wherein the amount of dispersing agent is 5 to 50% by weight of total composition, preferably it is 15 to 40% by weight of total composition.

4. The orally disintegrating composition according to claims 1 to 3, wherein the dispersing agent is selected from the group comprising calcium silicate, magnesium silicate, magnesium aluminium trisilicate, amorphous silica, magnesium carbonate heavy and the like and mixtures thereof.

5. The orally disintegrating composition according to claim 4, wherein the dispersing agent is preferably calcium silicate.

6. The orally disintegrating composition according to claim 5, wherein the calcium silicate has a median particle size of less than 50 micron, preferably less than 20 micron, more preferably less than 10 micron.

7. The orally disintegrating composition according to any preceding claims, wherein pramipexole is in the form of dihydrochloride monohydrate salt.

8. The orally disintegrating composition according to any preceding claims, wherein pramipexole dihydrochloride monohydrate is present in an amount of 0.01 to 5 % by weight of total composition, preferably it is 0.1 to 2.0 % by weight of total composition.

9. The orally disintegrating composition of any preceding claims, wherein said composition is in the form of tablets, sachets, minitablets, multilayer and multicoated tablets, pellets or powders; preferably said composition is in the form of tablet.

10. The orally disintegrating tablet composition according to any preceding claims, wherein the hardness of the tablet is between 5 N to 100 N, preferably it is between 20 N to 45 N.

11. The orally disintegrating tablet composition according to any preceding claims, wherein the firiabilite of the tablet is less than 1.0%.

12. The orally disintegrating tablet composition according to any preceding claims, wherein at least one pharmaceutically acceptable excipient is selected from the group comprising super disintegrants, diluents and/or fillers, binders and/or adhesives, lubricants, glidants, sweeteners, flavouring agents, coloring agents and preservatives.

13. The orally disintegrating tablet composition according to claim 14, wherein the super disintegrants are selected from the group comprising crospovidone, croscarmellose sodium, low-substituted hydroxypropylcellulose, sodium starch glycollate, pregelatinized starch and the like and mixtures thereof.

14. The orally disintegrating tablet composition according to claim 15, wherein the super disintegrant is preferably crospovidone.

15. The orally disintegrating tablet composition according to claim 16, wherein the amount of crospovidone is present in an amount of 0.1 to 30% by weight of total composition, preferably it is 1 to 15% by weight of total composition.

16. The orally disintegrating tablet composition according to any preceding claims, wherein the weight ratio of pramipexole dihydrochloride monohydrate to crospovidone is in the range of between 1:100 and 50:1 (w/w), preferably it is in the range of between 1:50 and 1:1 (w/w), more preferably it is in the range of between 1:45 and 1:20 (w/w).

17. The orally disintegrating tablet composition according to any preceding claims, wherein the weight ratio of crospovidone to calcium silicate is in the range of between 1:10 and 10:1 (w/w), preferably it is in the range of between 1:5 and 5:1 (w/w), more preferably it is in the range of between 1:5 and 1:1 (w/w).

18. The orally disintegrating tablet composition according to claim 14, wherein coloring agents are selected from the group comprising Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo carmine); iron oxides (such as; iron oxide red, yellow, black), quinoline yellow, flaming red, carmine, carmoisine, sunset yellow and the like and mixtures thereof; preferably the coloring agent is iron oxide yellow.

19. The orally disintegrating tablet composition according to any preceding claims comprising;
a. 0.01 to 5.0% by weight of pramipexole dihydrochloride monohydrate,
b. 5.0 to 90% by weight of mannitol,
c. 1.0 to 90% by weight of calcium silicate,
d. 0.1 to 25% by weight of polivinylpyrrolidone,
e. 1.0 to 30 % by weight of crospovidone,
f. 0.1 to 2% by weight of sucralose,
g. 0.1 to 5% by weight of orange flavour,
h. 0.1 to 10 % by weight of sodium stearyl fumarate.

20. A process for preparing the orally disintegrating tablet composition of any preceding claims, comprising the folowing steps;
a. dissolving pramipexole dihydrochloride monohydrate and polivinylpyrrolidone in water and/or alcohol to form an aqueous pramipexole dihydrochloride monohydrate solution,
b. while the solution is mixing, mannitol and half part of crospovidone is added and blended in a high-shear granulator to form granules,
c. sieving and drying the wet granules and sieving the dried granules,
d. adding calcium silicate, sucralose, the rest of the crospovidone and flavour and mixing them,
e. adding sodium stearyl fumarate to this mixture and blending them until obtaining a homogenous powder mixture,
f. compressing the blended mixture to form tablets.

21. A process for preparing the orally disintegrating tablet composition of any preceding claims, comprising the folowing steps;
a. dissolving pramipexole dihydrochloride monohydrate and polivinylpyrrolidone in water and/or alcohol to form an aqueous pramipexole dihydrochloride monohydrate solution,
b. mixing mannitol and half part of crospovidone in a high-shear granulator,
c. spraying the pramipexole dihydrochloride monohydrate solution to the blended powder mixture of mannitol and crospovidone ,
d. sieving and drying the wet granules and sieving the dried granules,
e. adding calcium silicate, sucralose, the rest of the crospovidone and flavour and mixing them,
f. adding sodium stearyl fumarate to this mixture and blending them until obtaining a homogenous powder mixture,
g. compressing the blended mixture to form tablets.

22. Use of the orally disintegrating composition of pramipexole dihydrochloride monohydrate of any preceding claims for preparing a pharmaceutical compositon for the treatment of Parkinsonism or parkinson's disease and complications or disorders, schizophrenia and restless leg syndrome.
